# NEW EUROPEAN PATENT SPECIFICATION

(11) **EP 0 991 416 B2**
(45) Date of publication and mention of the opposition decision: **31.10.2007**
(45) Mention of the grant of the patent: 09.10.2002
(21) Application number: 98945141.4
(22) Date of filing: 04.08.1998
(51) Int. Cl.: A61K 35/16

(54) **A UNIVERSALLY APPLICABLE BLOOD PLASMA**
UNIVERSELL VERWENDBARES BLUTTPLASMA
PLASMA SANGUIN UNIVERSEL

(30) Priority: 05.08.1997 EP 97113466
(43) Date of publication of application: 12.04.2000
(73) Proprietor: Octapharma AG, 8853 Lachen (CH)
(72) Inventor: MARGUERRE, Wolfgang, S-252 84 Helsingborg (SE); SVAE, Tor-Einar, N-2090 Hurdal (NO)
(74) Representative: Meyers, Hans-Wilhelm
(86) International application number: PCT/EP1998/004841
(87) International publication number: WO 1999/007390

(56) References cited:
- EP-A- 0 572 194
- WO-A1-91/14439
- US-A- 4 664 913
- R. BERKOW ET AL. (ED.'S): "THE MERCK MANUAL OF DIAGNOSIS AND THERAPY, FIFTEENTH EDITION" 1987 , MERCK SHARP & DOHME RESEARCH LABORATORIES , RAHWAY, N.J. XP002054540 see page 1132, line 23 - line 25
- E.F. Aubert et al., "The Agglutinin-Hinhibiting Substance in Human serum", J. Path. Bact. (1942) 54, S 89-104
- Sidney O. Levinson et al., "Suppression of Iso-Agglutinins" in J. Amer. Med. Assoc., 114, S. 2097-2098 (1940)
- Leitfaden des Transfusionswesens, VEB Verlag Volk und Gesundheit Berlin 1977, S. 115-116, Keck et al.

## Description

The present invention is related to a universally applicable blood plasma as well as a process for preparing same.

Blood plasma is a very widely used substitute for blood losses, for example, during operation or when severe bleedings occur after accidents. Since there are four major blood groups, at present different plasmas are prepared to serve patients with the different blood groups. Obviously, this is awkward since four different blood plasma preparations have to be stored by the respective blood banks or blood centers in the hospital. Furthermore, it would be necessary to determine the blood group of the patient who is in need of a blood substitute. This delay might be critical in case of emergency.

Aubert et al. (J. Path. Bact., 1942, 54, 89- 104) study the agglutinin-inhibiting substance in human serum under varying conditions.

The authors disclose a mixture of 2 to 5 parts of plasma of blood group A with 1 part of plasma of blood group B.

Thus, it is one object of the invention to provide a blood plasma preparation which can be applied universally to patients with different blood groups.

The present invention provides a universally applicable blood plasma obtainable by mixing blood or blood plasma of blood groups A and B and optionally blood or blood plasma derived from blood group AB, without admixing substantial amounts of blood or blood plasma derived from blood group O according to claim 1.

The invention further provides the use of a universally applicable blood plasma according to claim 9.

The blood plasma preparation of the invention is advantageous since there will be no risk of incompatible plasma infusions which may cause severe adverse reactions which can even be lethal. Additionally, the blood plasma preparation of the invention can be located at the site of the intended use, i. e. operation rooms and emergency rooms. Hence, the end user can have access to this lifesaving product immediately on request. The respective end user does not have to wait until the product ordered from the blood centers is released. At present the blood centers have to release a blood group specific plasma according to the blood group of the recipient.

In a preferred embodiment of the present invention the ABO blood group specific antibodies of the blood plasma are neutralized and/or removed.

A blood plasma preparation which is substantially free of fractions of group O leads to a more universally applicable blood plasma preparation. The blood plasma of the invention comprises preferably high amounts of blood plasma derived from donors having the blood group A, medium amounts of blood plasma derived from donors having the blood group B and optionally low amounts of blood plasma derived from donors having the blood group AB. The blood plasma of the invention is substantially free of blood plasma from donors having the blood group O. An embodiment of the present invention is a blood plasma comprising 6 to 10 parts of blood plasma derived from donors having the blood group A, 1 to 3 parts of blood plasma derived from donors having the blood group B, and 0.0 to 1.5 parts of blood plasma derived from donors having the blood group AB and substantially no blood plasma derived from blood group O.

In a very preferred embodiment of the present invention the blood plasma comprises 7.5 to 8.5 parts of blood plasma derived from donors having the blood group A, 1.5 to 2.5 parts of blood plasma derived from donors having the blood group B, and optionally about 1 part of blood plasma derived from donors having the blood group AB and substantially no blood or blood plasma derived from blood group O.

Preferably, the blood plasma of the invention has been prepared by pooled plasma derived from any donors. The pooled plasma preferably has been virus inactivated. The virus inactivation can be performed prior to mixing blood or blood plasma of different blood groups A, B and AB or after preparing of the blood plasma of the present invention. For virus inactivation any methods of the art can be used, for example, virus inactivation by irradiation with actinic radiation, pasteurization, solvent detergent treatment or combinations of the method. A well known method in the art, for example, is the solvent detergent treatment as disclosed in EP-A-0 131 740 as well as the method according to WO-A-94/17834 developed by Octapharma AG, Switzerland.

The blood plasma of the invention can be stored and delivered in any state known to the skilled person. The blood plasma may contain pharmaceutically acceptable·adjuvants, such as stabilizers and anticoagulants.

Preferably, the blood plasma of the invention is stored or delivered in a solid state, for example, in frozen form. Furthermore, it may be advantageous to store or deliver the blood plasma of the invention in a lyophilized or spray-dried form. In case the dried plasma is needed it can easily be dissolved in sterile water in order to infuse it in the patient.

The ABO blood group specific antibody titre of the blood plasma of the invention is preferably lower than 16 for anti A/anti B IgM and 64 for anti A/anti B IgG. In a very preferred embodiment the titre of the anti-A and anti-B antibodies is lower than 8 for IgM and lower than 32 for IgG.

The process for preparing the blood plasma of the invention comprises the steps of pooling blood or blood plasma of donors having the blood groups A, B and optionally AB as well as neutralizing and/or removing antibodies.

If blood is used as a starting material, blood plasma is produced from the blood pool by methods known in the art.

More than two-thirds of all blood donors have free A and/or B substance in plasma. These substances are almost identical to A and B antigenes bound to the surface of red blood cells. By mixing appropriate amounts of blood or blood plasma of the blood groups A, B and optionally AB anti-A and anti-B antibodies of subclasses IgM and IgG are neutralized by binding two free A and/or B substances and/or are removed during the further processing.

Surprisingly, although the plasma of the present invention used as raw material contains both residual red blood cells and the complete complement systems there are no signs of complement activation during the production or in the final product. According to the manufacturing process it is preferred that the mixing takes place during pooling of the plasma units in the beginning of the process combined with a complete cell removal and a virus inactivation process, preferably a solvent detergent treatment. The final product can be used without limitation on the infusion rate and total dosage.

The blood plasma of the invention prepared according to the process of the invention is advantageous since it additionally is coagulation active.

The present invention is further illustrated but not limited by the following example.

### Example

278 l of fresh-frozen plasma derived from blood group A, 68 l of B and 34 1 of AB are mixed together and allowed to thaw. Sodium dihydrogenphosphate dihydrate is added as a buffer to stabilize the plasma proteins. After filtration through a membrane having a pore size of 1 µm, the obtained fraction is virus inactivated by the solvent detergent method. After removal of the virus inactivating agents, glycine is added to adjust the osmolarity. During qualitiy control the amount of free anti-A and anti-B antibodies is tested. Such tests are well-known in the art. The titre of anti-A and anti-B antibodies should be < 8 for IgM and < 32 for IgG.

## Claims

1. A universally applicable blood plasma obtainable by a process comprising the steps of mixing blood or blood plasma of blood groups A and B and optionally blood or blood plasma of blood group AB without admixing substantial amounts of blood or blood plasma derived from blood group 0, **characterized in that** are mixed
- 6 to 10 parts of blood or blood plasma derived from donors having the blood group A;
- 1 to 3 parts of blood or blood plasma derived from donors having the blood group B
- 0.0 to 1.5 parts of blood or blood plasma derived from donors having the blood group AB,
- substantial no blood or blood plasma derived from donors having the blood group 0.

2. The blood plasma of claim 1, wherein the AB0 blood group specific antibodies of the blood plasma are neutralized and/or removed.

3. The blood plasma of any one of the foregoing claims in liquid, frozen, or dried state.

4. The blood plasma of claim 3 in lyophilized or spray-dried form.

5. The blood or blood plasma of any one of the foregoing claims comprising pharmaceutically acceptable adjuvants, such as stabilizers and anticoagulants.

6. The blood or blood plasma of any one of the foregoing claims which blood plasma was subjected to a treatment for virus inactivation.

7. A process of preparing the blood or blood plasma of claim 1 and 2 comprising the steps of
- pooling 6 to 10 parts of blood or blood plasma derived from donors having the blood group A,
- 1 to 3 parts of blood or blood plasma derived from donors having the blood group B
- 0.0 to 1.5 parts of blood or blood plasma derived from donors having the blood group AB,
- substantially no blood or blood plasma derived from donors having the blood group 0, and
- neutralizing and/or removing AB0 blood group specific antibodies.

8. The process of claim 7 further comprising the step of spray-drying or lyophilizing the plasma.

9. The use of a blood plasma for the preparation of a medicament which is a universally applicable blood plasma, wherein the blood plasma is obtainable by a process comprising the steps of mixing blood or blood plasma of blood groups A and B and blood or blood plasma of blood group AB without admixing substantial amounts of blood or blood plasma derived from blood group O.

10. The use of claim 9, wherein the ABO blood group specific antibodies of the blood plasma are neutralized and/or removed.

11. The use of claim 10, wherein the blood plasma comprises high amounts of blood plasma derived from donors having the blood group A, medium amounts of blood plasma derived from donors having the blood group B, and optionally low amounts of blood plasma derived from donors having the blood group AB.

12. The use of claim 11, wherein the blood plasma comprises
- 6 to 10 parts of blood or blood plasma derived from donors having the blood group A,
- 1 to 3 parts of blood or blood plasma derived from donors having the blood group B,
- 0.0 to 1.5 parts of blood or blood plasma derived from donors having the blood group AB,
- substantially no blood or blood plasma derived from donors having the blood group O.

13. The use of any of the foregoing claims, wherein the blood plasma is in liquid, frozen, or dried state.

14. The use of claim 13 wherein the blood plasma is in lyophilized or spray-dried form.

15. The use of any of the foregoing claims, wherein the blood or blood plasma comprises pharmaceutically acceptable adjuvants, such as stabilizers and anticoagulants.

16. The use of any of the foregoing claims, wherein the blood or blood plasma was subjected to a treatment for virus inactivation.

## Patentansprüche

1. Universell anwendbares Blutplasma, das nach einem Verfahren erhältlich ist, das die Schritte des Mischens von Blut oder Blutplasma der Blutgruppen A und B sowie gegebenenfalls Blut oder Blutplasma der Blutgruppe AB umfasst, ohne wesentliche Mengen von Blut oder Blutplasma, das von der Blutgruppe 0 abgeleitet ist, beizumischen, **dadurch gekennzeichnet, dass** Folgendes miteinander gemischt wird:
- 6 bis 10 Teile Blut oder Blutplasma, das von Spendern mit der Blutgruppe A stammt;
- 1 bis 3 Teile Blut oder Blutplasma, das von Spendern mit der Blutgruppe B stammt;
- 0,0 bis 1,5 Teile Blut oder Blutplasma, das von Spendern mit der Blutgruppe AB stammt;
- im Wesentlichen kein Blut oder Blutplasma, das von Spendern mit der Blutgruppe 0 stammt.

2. Blutplasma gemäß Anspruch 1, wobei die AB0-blutgruppenspezifischen Antikörper des Blutplasmas neutralisiert und/oder entfernt sind.

3. Blutplasma gemäß einem der vorstehenden Ansprüche in flüssigem, gefrorenem oder getrocknetem Zustand.

4. Blutplasma gemäß Anspruch 3 in lyophilisierter oder sprühgetrockneter Form.

5. Blut oder Blutplasma gemäß einem der vorstehenden Ansprüche, das pharmazeutisch annehmbare Adjuvantien, wie Stabilisatoren und Antikoagulantien, umfasst.

6. Blut oder Blutplasma gemäß einem der vorstehenden Ansprüche, wobei das Blutplasma einer Behandlung zur Virusinaktivierung unterzogen wurde.

7. Verfahren zur Herstellung des Blutes oder Blutplasmas gemäß Anspruch 1 und 2, umfassend die folgenden Schritte:
- Zusammenschütten von 6 bis 10 Teilen Blut oder Blutplasma, das von Spendern mit der Blutgruppe A stammt;
- 1 bis 3 Teilen Blut oder Blutplasma, das von Spendern mit der Blutgruppe B stammt;
- 0,0 bis 1,5 Teilen Blut oder Blutplasma, das von Spendern mit der Blutgruppe AB stammt;
- im Wesentlichen keinem Blut oder Blutplasma, das von Spendern mit der Blutgruppe 0 stammt; und
- Neutralisieren und/oder Entfernen von AB0-blutgruppenspezifischen Antikörpern.

8. Verfahren gemäß Anspruch 7, das weiterhin den Schritt des Sprühtrocknens oder Lyophilisierens des Plasmas umfasst.

9. Verwendung eines Blutplasmas zur Herstellung eines Medikaments, bei dem es sich um ein universell anwendbares Blutplasma handelt, wobei das Blutplasma nach einem Verfahren erhältlich ist, das die Schritte des Mischens von Blut oder Blutplasma der Blutgruppen A und B sowie Blut oder Blutplasma der Blutgruppe AB umfasst, ohne wesentliche Mengen von Blut oder Blutplasma, das von der Blutgruppe 0 abgeleitet ist, beizumischen.

10. Verwendung gemäß Anspruch 9, wobei die AB0-blutgruppenspezifischen Antikörper des Blutplasmas neutralisiert und/oder entfernt sind.

11. Verwendung gemäß Anspruch 10, wobei das Blutplasma große Mengen an Blutplasma, das von Spendern mit der Blutgruppe A stammt, mittlere Mengen an Blutplasma, das von Spendern mit der Blutgruppe B stammt, und gegebenenfalls geringe Mengen an Blutplasma, das von Spendern mit der Blutgruppe AB stammt, umfasst.

12. Verwendung gemäß Anspruch 11, wobei das Blutplasma Folgendes umfasst:
- 6 bis 10 Teile Blut oder Blutplasma, das von Spendern mit der Blutgruppe A stammt;
- 1 bis 3 Teile Blut oder Blutplasma, das von Spendern mit der Blutgruppe B stammt;
- 0,0 bis 1,5 Teile Blut oder Blutplasma, das von Spendern mit der Blutgruppe AB stammt;
- im Wesentlichen kein Blut oder Blutplasma, das von Spendern mit der Blutgruppe 0 stammt.

13. Verwendung gemäß einem der vorstehenden Ansprüche, wobei das Blutplasma in flüssigem, gefrorenem oder getrocknetem Zustand vorliegt.

14. Verwendung gemäß Anspruch 13, wobei das Blutplasma in lyophilisierter oder sprühgetrockneter Form vorliegt.

15. Verwendung gemäß einem der vorstehenden Ansprüche, wobei das Blut oder Blutplasma pharmazeutisch annehmbare Adjuvantien, wie Stabilisatoren und Antikoagulantien, umfasst.

16. Verwendung gemäß einem der vorstehenden Ansprüche, wobei das Blut oder Blutplasma einer Behandlung zur Virusinaktivierung unterzogen wurde.

## Revendications

1. Plasma sanguin applicable universellement, susceptible d'être obtenu par un procédé comprenant les étapes consistant à mélanger du sang ou du plasma sanguin des groupes A et B et éventuellement du sang ou du plasma sanguin du groupe sanguin AB sans mélanger des quantités substantielles de sang ou de plasma sanguin issu du groupe sanguin O, **caractérisé en ce que** l'on mélange
- 6 à 10 parties de sang ou de plasma sanguin issu de donneurs ayant le groupe sanguin A,
- 1 à 3 parties de sang ou de plasma sanguin issu de donneurs ayant le groupe sanguin B
- 0,0 à 1,5 partie de sang ou de plasma sanguin issu de donneurs ayant le groupe sanguin AB,
- essentiellement pas de sang ou de plasma sanguin issu de donneurs ayant le groupe sanguin O.

2. Plasma sanguin selon la revendication 1, dans lequel les anticorps spécifiques aux groupes sanguins ABO du plasma sanguin sont neutralisés et/ou éliminés.

3. Plasma sanguin selon l'une quelconque des revendications précédentes, à l'état liquide, congelé ou séché.

4. Plasma sanguin selon la revendication 3, sous forme lyophilisée ou séchée par pulvérisation.

5. Sang ou plasma sanguin selon l'une quelconque des revendications précédentes, comprenant des adjuvants pharmaceutiquement acceptables, tels que des stabilisants et des anticoagulants.

6. Sang ou plasma sanguin selon l'une quelconque des revendications, lequel plasma sanguin a été soumis à un traitement d'inactivation de virus.

7. Procédé de préparation de sang ou de plasma sanguin selon les revendications 1 et 2, comprenant les étapes consistant
- à regrouper 6 à 10 parties de sang ou de plasma sanguin issu de donneurs ayant le groupe sanguin A,
- 1 à 3 parties de sang ou de plasma sanguin issu de donneurs ayant le groupe sanguin B
- 0,0 à 1,5 partie de sang ou de plasma sanguin issu de donneurs ayant le groupe sanguin AB,
- essentiellement pas de sang ou de plasma sanguin issu de donneurs ayant le groupe sanguin O, et
- à neutraliser et/ou éliminer les anticorps spécifiques aux groupes sanguins ABO.

8. Procédé selon la revendication 7, comprenant en outre l'étape consistant à sécher par pulvérisation ou à lyophiliser le plasma.

9. Utilisation d'un plasma sanguin pour la préparation d'un médicament qui est un plasma sanguin applicable universellement, dans laquelle le plasma sanguin est susceptible d'être obtenu par un procédé comprenant les étapes consistant à mélanger du sang ou du plasma sanguin des groupes sanguins A et B et du sang ou du plasma sanguin du groupe sanguin AB sans mélanger des quantités substantielles de sang ou de plasma sanguin issu du groupe sanguin O.

10. Utilisation selon la revendication 9, dans laquelle les anticorps spécifiques aux groupes sanguins ABO du .plasma sanguin sont neutralisés et/ou éliminés.

11. Utilisation selon la revendication 10, dans laquelle le plasma sanguin comprend des quantités élevées de plasma sanguin issu de donneurs ayant le groupe sanguin A, des quantités moyennes de plasma sanguin issu de donneurs ayant le groupe sanguin B et facultativement de faibles quantités de plasma sanguin issu de donneurs ayant le groupe sanguin AB.

12. Utilisation selon la revendication 11, dans laquelle le plasma sanguin comprend
- 6 à 10 parties de sang ou de plasma sanguin issu de donneurs ayant le groupe sanguin A,
- 1 à 3 parties de sang ou de plasma sanguin issu de donneurs ayant le groupe sanguin B,
- 0,0 à 1,5 partie de sang ou de plasma sanguin issu de donneurs ayant le groupe sanguin AB,
- essentiellement pas de sang ou de plasma sanguin issu de donneurs ayant le groupe sanguin O.

13. Utilisation selon l'une quelconque des revendications précédentes, dans laquelle le plasma sanguin est à l'état liquide, congelé ou séché.

14. Utilisation selon la revendication 13, dans laquelle le plasma sanguin est sous forme lyophilisée ou séchée par pulvérisation.

15. Utilisation selon l'une quelconque des revendications précédentes, dans laquelle le sang ou le plasma sanguin comprend des adjuvants pharmaceutiquement acceptables, tels que des stabilisants et des anticoagulants.

16. Utilisation selon l'une quelconque des revendications précédentes, dans laquelle le sang ou le plasma sanguin a été soumis à un traitement d'inactivation de virus.
